# EUROPEAN PATENT SPECIFICATION

(11) **EP 4 175 528 B1**
(45) Date of publication and mention of the grant of the patent: **25.09.2024**
(21) Application number: 21740437.5
(22) Date of filing: 29.06.2021
(51) Int. Cl.: A61B 1/00, H05K 9/00

(54) **RF SHIELDING BOX, MEDICAL INSTRUMENT AND METHOD OF FIXING**
HF-ABSCHIRMUNGSBOX, MEDIZINISCHES INSTRUMENT UND VERFAHREN ZUR BEFESTIGUNG
BOÎTE DE BLINDAGE RF, INSTRUMENT MÉDICAL ET PROCÉDÉ DE FIXATION

(30) Priority: 01.07.2020 DE 102020117363
(43) Date of publication of application: 10.05.2023
(73) Proprietor: Karl Storz SE & Co. KG, 78532 Tuttlingen (DE)
(72) Inventor: POLLUKS, Indrek-Toomas, 78532 Tuttlingen Baden-Württemberg (DE)
(86) International application number: PCT/EP2021/067827
(87) International publication number: WO 2022/002925

(56) References cited:
- EP-B1- 2 641 542
- EP-B1- 3 257 429
- WO-A1-2013/111665
- CN-A- 1 145 573
- JP-A- 2017 006 162
- JP-A- H0 836 139

## Description

The invention related to a RF shielding box, a medical instrument comprising such a shielding box and a method for fixing such a RF shielding box inside a connector for such a medical instrument.

A RF shielding box is understood to be a box, preferably made from metal, which prevents radiofrequency radiation from entering the box. Inside the box, usually sensitive electronics is placed.

In the prior art endoscopes are known. An endoscope apparatus may have an insertion portion as an elongated long member which is inserted into a site to be observed, e.g. a lumen of a large intestine or the like, an operation section which is connectively provided at a proximal end portion of the insertion portion, a universal cable which is a composite cable provided extensively from a side surface of the operation section, and an endoscope connector, which hereinafter may simply be abbreviated as a connector, which is provided at an end portion of the universal cable and is a connector for a medical instrument detachably connected to an external device which is a video processor integrated with a light source apparatus.

In the prior art, it is also known that inside such an endoscope connector shielding members may be used.

Connectors used in gastroenterology endoscopes generally have ducts for feeding water, air or gas and a suction channel. Connectors in pulmonology or urology endoscopes generally do not provide water, air or gas.

In the prior art, it is known that a shielding box inside the connector may be fixed to a mechanical holding structure by using soldering joints. This has the disadvantage, that a disassembling and assembling such a feeling box is tedious and takes a lot of time.

EP 3 257 429 B1 discloses an endoscope comprising a shield box for electrical connections, wherein the shielding box features a conical box shape. The shielding box is mounted in the endoscope partly on face surfaces, using additional parts.

JP 2017-6162 A disclose a RF shielding box for use inside of an connector for a medical instrument, in particular an endoscope, wherein the shielding box is cylinder-shaped, is conically tapered, wherein both a first end face an da second end face of the shielding box are rectangular shaped. Mounting of the shielding box is enabled by additional parts. A further RF shielding box is disclosed in EP 2 641 542 B1.

An object of the invention is to improve the prior art. In particular, it is an object of the invention to provide a device for mounting a shielding box inside an endoscope connector in an easy way, in particular an easily exchangeable way. In particular, it is an object of the invention that the shielding box should be mountable inside the connector without regular fastening elements, like e.g. screws or the like.

This object of the invention is solved by a RF shielding box for use inside a connector for a medical instrument having the features of independent claim 1. Preferred embodiments of the invention are disclosed in the sub claims.

A "cylinder" is understood to be a three-dimensional shape bounded by a cylindrical surface and two parallel planes. The line segments determined by an element of the cylindrical surface between the two parallel planes is called an element of the cylinder. All the elements of a cylinder have equal lengths. The region bounded by the cylindrical surface in either of the parallel planes is called a base of the cylinder. The two bases are preferably identical figures or shapes. If the elements of the cylinder are perpendicular to the planes containing the bases, the cylinder is a right cylinder; otherwise it is called an oblique cylinder. Preferably, the cylinder is a right cylinder.

A "conically tapered area" is understood to be a two-dimensional area whose outer diameter is conical and decreases from one end to another end.

It is understood that an "area of the first end face" is larger than the "area of the second end face". A "longitudinal axis" of the shielding box preferably is a middle axis from a central point on the first end face to a central point on the second end face.

A "rectangular shaped plate" may either be an integral part of the first end face or the second end face, wherein at least a part of the plate protrudes from the area of the first end face or the second end face, wherein the area is part of the cylinder. A rectangular shaped plate may also be attached to the first end face or the second end face, wherein at least a part of the plate protrudes from the area of the first end face or the second end face, wherein the area is part of the cylinder.

The plates of the first end face of the shielding box are parallel to each other. The plates of the second end face of the shielding box are parallel to each other.

The "protruding parts" of the plates are adapted and installed such that the shielding box can be mounted inside the connector. This may e.g. be achieved by attaching the shielding box to some fixed mounting structure which is inside the connector and fixed with respect to or at the connector, wherein the protruding part of plates engage into mechanical structures, e.g. holes of a predetermined shape and spatial arrangement such that when the gravitational force pulls the shielding box to the gravitational centre of the earth, then the engagement between the protruding parts and the mechanical structure is such that the mechanical structure exerts a mounting force onto the shielding box, such that the shielding box does not move anymore and is in a stable position.

The base area of the shielding box is understood to be on the bottom of the shielding box, when the shielding box is installed on the rails, i.e. in the installation position. The installation position or the installation arrangement of the shielding box is preferably such that the longitudinal axis of the shielding box is horizontal. This has the advantage, that a user installing the shielding box can access the shielding box and the rails in a practical and easy way. The shielding box is preferably oriented such that the base area is horizontal. The base area may be a cutting plate or a stamped part having at least one bent edge, preferably two bent edges. Preferably the bent edges form the "protruding parts" of the plates.

Preferably, both the first end face and the second end face of the shielding box each comprise two rectangular shaped plates protruding from the area of the first end face and the second end face, where the plates are parallel to the first end face or the second end face.

When being installed, the first end face and the second end face of the shielding box preferably are vertical.

When being installed, the conically tapered faces of the shielding box preferably are vertical.

An advantage of the RF shielding box according to the present invention is that it can be disassembled and assembled inside the connector very easily and fast. Another advantage is, that for mounting the shielding box only parts are used, which are resistant to corrosion. Yet another advantage is that the fixation of their shielding box is stable and robust.

According to a preferred embodiment of the invention, longitudinal axes of the plates are each parallel to the base area of the cylinder. This has the advantage, that the plates protrude vertically from the first end face and the second end face, which is very convenient for the shielding box to be mounted inside the connector, e.g. to a structural element.

Preferably, the longitudinal axes of the plates lie within a single plane which is parallel to the base area of the cylinder. This has the advantage, that the plates are arranged at the same height of the shielding box, when the shielding box is used as intended.

According to another embodiment the base area is an isosceles trapezoid. In Euclidean geometry, an isosceles trapezoid is a convex quadrilateral with a line of symmetry bisecting one pair of opposite sides. In any isosceles trapezoid, two opposite sides, i.e. the bases, are parallel, and the two other sides, the legs, are of equal length. Here, the bases of the isosceles trapezoid are situated at the first and the second end of the shielding box.

This has the advantage, that the shielding box has the same symmetry as the isosceles trapezoid, i.e. the shielding box is symmetrical with respect to the longitudinal axis from the first end face to the second end face.

The base area and the two rectangular shaped plates may be part of a single workpiece which may be manufactured by bending a cutting plate at at least one edge. Preferably the cutting plate is bent along two preferably parallel edges at opposite ends of the cutting plate. Two opposing two rectangular shaped plates on one end of the cutting plate preferably lie on a straight line. The two opposing two rectangular shaped plates on the other end of the cutting plate preferably lie on a straight line, too. Preferably, these two straight lines are parallel to each other and are preferably parallel to the bent edges.

According to the invention, at least two of the protruding parts of the plates are adapted and installed to be arranged inside at least two holes, wherein the holes are arranged inside a first and a second rail, which each when used as intended are parallel to the conically tapered faces of the shielding box.

The first and the second rail are used to mount or fix the shielding box to the rails. Preferably, the holes, into which the protruding parts of the plates engage, extent horizontally inside the rails, when the rails are used in the installation arrangement.

The first and the second rail are arranged on opposing sides of the shielding box.

The at least two holes preferably comprise for instance two holes or four holes. In the case of two holes, it is preferred, that the first hole is arranged at the first end of the first rail and the second hole is arranged at the first end of the second rail.

In the case of four holes, it is preferred, that the first hole is arranged at the first end of the first rail, the second hole is arranged at the first end of the second rail, the third is arranged at the second end of the first rail, and the fourth hole is arranged at the second end of the second rail.

In particular for the case of two holes, it is further preferred, that the first rail and / or the second rail each comprise another hole in the inside of the rail, such that two opposing plates can be moved or shifted back and forth inside the holes.

According to the invention, the RF shielding box further comprises a first rail and a second rail, which each are parallel to the conically tapered faces of the shielding box. Here the first rail and the second rail each comprise a hole on a first end and/or a second end. Each rail comprises for instance one or preferably two holes. Further the holes on the first end of the first rail and the second rail are adjacent to the first end face of the shielding box. The holes at the second end of the first rail and the second rail, if existent, are adjacent to the second end face of the shielding box. This has the advantage, that the holes are arranged that the protruding parts of the RF shielding box can easily be inserted into the holes.

The two protruding parts of the first end face may be arranged inside the holes on the first end of the first rail and the second rail.

Additionally or alternatively, the two protruding parts of the second end face may be arranged inside the holes on the second end of the first rail and the second rail.

This has the advantage, that the shielding box is securely tightened to the first and second rail, which are arranged to either side of the shielding box.

Preferably the holes are slits, which preferably are aligned vertically, when the shielding box is used in the installation arrangement. The size of the slit preferably is such that it is slightly larger than the size or the cross-section of the respective plate to be arranged inside the slit.

This has the advantage, that the plates are securely tightened in the slits, which in turn achieves that the shielding box is securely tightened to the first and second rail.

The shielding box may be adapted and installed to be press fitted between the first and the second rail such that it is guaranteed that assembled parts inside the shielding box do not move or are shaken.

The first end of the first rail and the first end of the second rail may be adapted to be fixed to opposite ends on an inside of a casing element of the connector or to opposite ends or opposite positions on an outer rim of an opening of the casing element of the connector.

Additionally or alternatively, the second end of the first rail and the second end of the second rail may be adapted to be fixed to opposite ends on the outside of an end side fixation device of a cable which is connectable to the connector. The fixation device of a cable may e.g. be a bushing, a socket, a plug socket or a connector, in particular a female connector.

The opposite ends on an inside of a casing element of the connector or to opposite ends or opposite positions on an outer rim of an opening of the casing element of the connector on the one hand and the opposing ends on the outside of the end side fixation device on the other hand may lie within a plane.

Preferably, the diameter of the outer rim of an opening of the casing element is larger than the outside of the end side fixation device. The opening of the casing element may have a roundish or round shape.

This has the advantage that all devices or parts used to fix the shielding box lie within a single plane, which preferably is horizontal, when used in the installation arrangement.

The press fit may be achieved by bending the first end of the first rail towards the second rail and then fixing the first end of the first rail to an inside of a casing element and/or by bending the first end of the second rail towards the first rail and then fixing the first end of the second rail to an inside of the casing element. This bending advantageously has an elastic effect.

According to some embodiments, the more rails ends are bent to the inside, the easier it is to attach them to the casing member. Preferably, the distance between the casing element and the fixation points is chosen such that that there will be a press fit.

Alternatively, the press fit may be achieved by fixing the second end of the first rail and the second end of the second rail to opposing ends on the outside of the end side fixation device such that the first rail and second rail are pressed against the conically tapered faces of the shielding box. This has the advantage, that the press fit may be achieved by only tightening the screws on the second end of the rails.

According to another aspect of the invention, the object of the invention is solved by a medical instrument, in particular an endoscope, comprising a shielding box which was described above.

According to yet another aspect of the invention, the object of the invention is solved by a method for fixing a RF shielding box inside a connector for a medical instrument, in particular an endoscope, the shielding box preferably being an abovementioned shielding box.

According to a first step of the method a second end of a first rail and a second end of a second rail are fixed to opposing ends on an outside of an end side fixation device of a cable which is connectable to the connector.

According to a second step of the method the shielding box is guided such that plates of a second end face run inside of elongated holes of both the first rail and the second rail until the plates contact an end of the elongated holes near a second end of the first rail or second rail.

According to a third step tubes, e.g. water supply tubes, which run from the end side fixation device of the cable towards the casing member or vice versa, are connected. This step may also be performed before the first step or after the last step of the present method.

According to a fourth step a casing element of the connector is provided such that opposing ends of an outer rim of an opening of the casing element are adjacent to a first end of the first rail and a first end of the second rail.

According to a fifth step the first end of the first rail and the first end of the second rail are fixed to the opposing ends on the outer rim of the opening of the casing element of the connector.

An embodiment of the invention is shown in the drawings and will be explained in more detail in the following description.

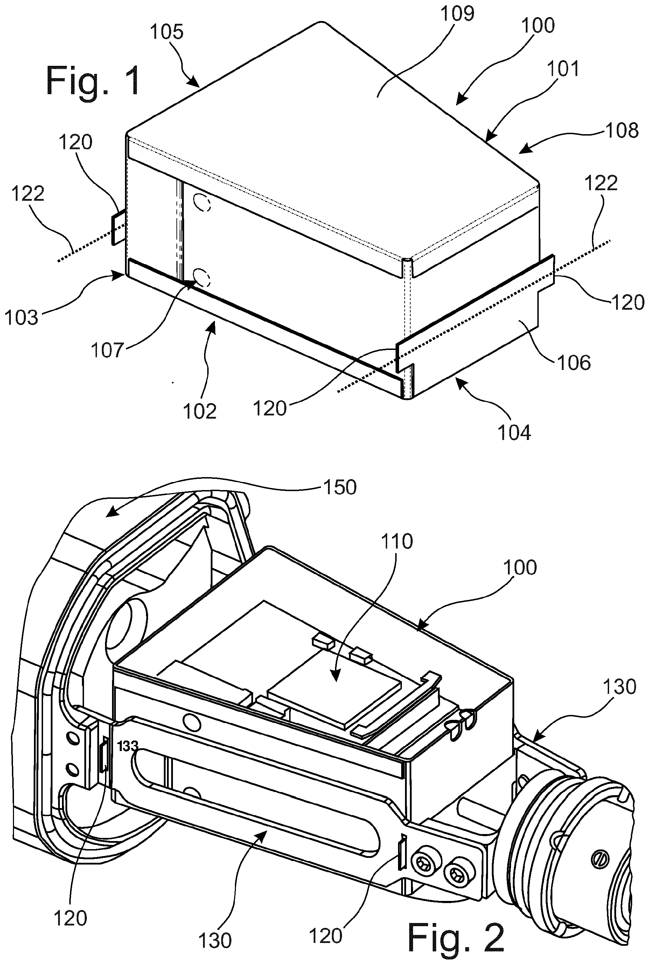

- Figure 1: shows a shielding box with four rectangular shaped plates according to a first embodiment of the invention.
- Figure 2: shows a shielding box according to a second embodiment of the invention, wherein the shielding box is installed between two rails, wherein the rails are fixed to both a casing element and a bushing.

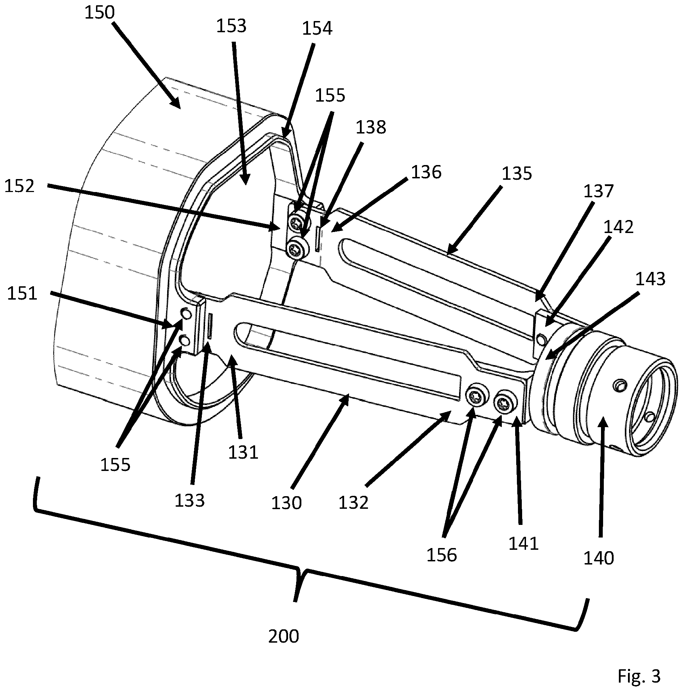

- Figure 3: shows how two rails are fixed to both the casing element and the bushing, wherein the rails are different from the rails shown in the second embodiment of the invention in figure 2; the rails are part of a third embodiment of the invention.

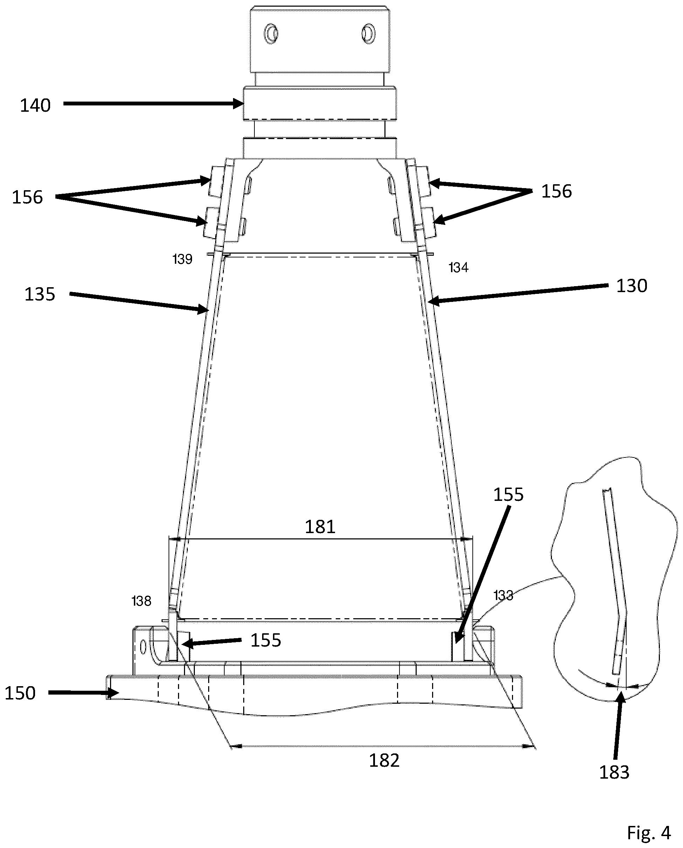

- Figure 4: shows the shielding box with the two rails and which are fixed to both the casing element and the bushing as seen from a top view. The embodiment of figure 4 is the same as in figure 2, i.e. the second embodiment.
- Figure 5: shows an endoscope 501 comprising a shielding box in a connector 503.
- Figure 6: shows a flow chart for a method for fixing a RF shielding box inside a connector for a medical instrument according to an embodiment of the invention.

A RF shielding box 100 is cylinder-shaped and has a base area 102. Opposite of the base area 102, the cylinder 101 comprises a surface 109, which has the same dimension as the base area 102. Both the base area 102 and the surface 109 are conically tapered from a first end 103 of the shielding box 100 to a second end 104 of the shielding box 100. Both the base area 102 and the surface 109 have the shape of an isosceles trapezoid.

At the first end 103 of the shielding box 100 a first end face 105 is arranged and at the second end 104 of the shielding box 100 a second end face 106 is arranged. Both the first end face 105 and the second end face 106 of the shielding box 100 are rectangular shaped.

Both the first end face 105 and the second end face 106 of the shielding box 100 each comprise two rectangular shaped plates 120 protruding from the area of the first end face 105 and the second end face 106. The plates 120 are parallel to the first end face 105 or the second end face 106, respectively. The plates 120 of the first end face 105 and the second end face 106 of the shielding box 100 are parallel to each other, respectively. Longitudinal axes 122 of the plates 120 lie within a single plane which is parallel to the base area 102 or the surface 109 of the cylinder 101.

The protruding parts of the plates 120 are used to mount the shielding box 100 inside the connector 200.

The RF shielding box 100 is used inside a connector 200 for an endoscope. Inside the RF shielding box 100 electronics 110 may be mounted. The connector 200 comprises a casing element 150, a bushing 140 and a second casing element connecting the casing element 150 and the bushing 140. The RF shielding box 100 and the rails are surrounded by the second casing element. A cross-section of the second casing element starting from the casing element 150 towards the bushing 140 is as follows: at first the cross-section is octagonal, then the cross-section will get smaller and round at the end towards the bushing 140.

The connector 200 has an elongated shape with differing cross-section. The longitudinal axis of the connector 200 is horizontal, when the connector 200 and the shielding box 100 are used in the installation arrangement or installation position.

The connector 200 has a casing element 150 on a first side of the connector 200 and a bushing 140 for a universal cable on a second side of the connector 200. The bushing 140 and the casing element 150 are fixed to one another using a first rail 130 and the second rail 135, whose distance from each other is conically tapered from the casing element 150 towards the bushing 140.

The casing element 150 comprises an opening 153 facing the shielding box 100 and the bushing 140. The opening 153 has a roundish rim 154. The cross-section perpendicular to the longitudinal axis of the casing element 150 has the shape of an octagon. Accordingly, the opening 153 and the rim 154 also have the shape of an octagon. The octagon has rounded edges. The area of the opening 153 is slightly larger than the first end face 105. On opposite sides of the octagon, the rim 154 comprises a tab-like first reinforcement structure 151 and a tab-like second reinforcement structure 152. A connection line between the first reinforcement structure 151 and the second reinforcement structure 152 to is preferably horizontal, when the shielding box 100 is in the installation position.

The bushing 140 for the universal cable has a roundish shape which is slightly smaller than the area of the second end face 106. The bushing 140 is hollow such that ducts can be guided inside. At a rim 143 of the bushing 140, which faces the second end face 106 of the shielding box 100, the bushing 140 comprises a first longitudinal tab 141 and a second longitudinal tab 142 which are arranged on the rim 143 at opposite positions. In the installation position of the shielding box 100, the first longitudinal tab 141 and the second longitudinal tab 142 are horizontal. The first longitudinal tab 141 and the second longitudinal tab 142 are each parallel to the first rail 130 and the second rail 135.

A first end 131 of the first rail 130 is fixed to the first reinforcement structure 151 and a second end 132 of the first rail 130 is fixed to the first longitudinal tab 141. A first end 136 of the second rail 135 is fixed to the second reinforcement structure 152 and a second end 137 of the second rail 135 is fixed to the second longitudinal tab 142.

The first rail 130 and the second rail 135 each are parallel to the conically tapered faces 107, 108 of the shielding box 100.

According to the second and third embodiment, the first rail 130 comprise a slit like hole 133 on the first end 131 of the first rail 130 and the second rail 135 comprise a slit like hole 138 on the first end 136 of the second rail 135.

According to the third embodiment the first rail 130 comprise a slit like hole 134 on the second end 132 of the first rail 130 and the second rail 135 comprise a slit like hole 139 on the second end 137 of the second rail 135.

The first rail 130 and the second rail 135 each comprise a plate like elongated body which comprises an elongated hole in a central part of the rail. In the installation position or installation arrangement, that longitudinal axes of both the first rail 130 and the second grade 135 are horizontal and the second dimension of the plate like structure extends vertically.

At the first end 131 the first rail 130 is fixed to the tab-like first reinforcement structure 151 using two screws 155, wherein the first end 131 of the first rail 130 comprises two holes and the first reinforcement structure 151 comprises two threads. Here the threads and screws 155 are arranged vertically above one another. The first end 131 of the first rail 130 is fixed to the first reinforcement structure 151 from the inside. Therefore, the screws 155 are arranged on the inside of the first rail 130. At the first end 136 the second rail 135 is fixed to the tab-like second reinforcement structure 152 using another two screws 155, wherein the first end 136 of the second rail 135 comprises two holes and the second reinforcement structure 152 comprises two threads. Here the threads and screws 155 are arranged vertically above one another. The first end 136 of the second rail 130 is fixed to the second reinforcement structure 152 from the inside. Therefore, the screws 155 are arranged on the inside of the second rail 135.

The distance 182 between the inner ends of the first reinforcement structure 151 and the second reinforcement structure 152 is smaller than the distance 181 between the outer ends of the first rail 130 and the second rail 135 at the casing member 150 at rest. That means that the outer ends of the first rail 130 and the second rail 135 have to be bent to the inside in order to fit between the inner ends of the first reinforcement structure 151 and the second reinforcement structure 152.

Preferably, an end of first rail 130 and the second rail 135 at the casing member 150 is bent to the inside by a predetermined angle 183. When tightening the screws 155 to the first rail 130 and the second rail 135, the first rail 130 and the second rail 135 will be squeezed against the casing element 150 such that the bending of the ends of the first rail 130 and the second rail 135 is removed.

At the second end 132 the first rail 130 is fixed to the first longitudinal tab 141 using two screws 156, wherein the second end 132 of the first rail 130 comprises two holes and the first longitudinal tab 141 comprises two threads. Here the threads and screws 156 are arranged horizontally next to one another along the longitudinal axis of the first rail 130. The first rail 130 is fixed on the outer side of the first longitudinal tab 141. Therefore, the screws 156 are arranged on the outside of the first rail 130. At the second end 137 the second rail 135 is fixed to the second longitudinal tab 142 using another two screws 156, wherein the second end 137 of the second rail 135 comprises two holes and the second longitudinal tab 142 comprises two threads. Here the threads and screws 156 are arranged horizontally next to one another along the longitudinal axis of the second rail 135. The second rail 135 is fixed on the outer side of the second longitudinal tab 142. Therefore, the screws 156 are arranged on the outside of the second rail 135.

According to the third embodiment of the shielding box 100 and the rails 130, 135, the shielding box 100 may be installed as follows: First, the first rail 130 and the second rail 135 are fixed at the bushing 140. Second, the protruding parts of the plates 120 at the second and phase 106 of the shielding box 100 are inserted into the elongated holes inside the first rail 130 and the second rail 135. Third, the whole shielding box 100 is guided towards the bushing 140, such that the plates 120 contact the end of the elongated holes the other bushing 140. Fourth, the two protruding parts of the plates 120 of the first end face 105 are inserted into the holes 133 of the first rail 130 and the hole 138 of the second rail 135, respectively. Fifth, the first end 131 of the first rail 130 and the first end 136 of the second rail 135 are fixed to the first reinforcement structure 151 and the second reinforcement structure 152. After that the second casing member may be fixed to the casing member 150 and the bushing 140. Before that step tubes running from the bushing 140 towards the casing member 150 may also be connected.

The third embodiment has the advantage that in order to install the shielding box 100, there is no need to remove the screws 156 on the side of the bushing 140.

According to the second embodiment of the shielding box 100 and the rails 130, 135, the shielding box 100 may be installed as follows:

First, the first rail 130 and the second rail 135 are fixed to opposite conically tapered faces 107, 108 of the shielding box 100). This is done by moving the first rail 130 onto the conically tapered face 107 such that the protruding part of the plate 120 of the first end face 105 enters the slit like hole 133 of the first rail 130 and the protruding part of the plate 120 of the second end face 106 enters the slit like hole 134 of the first rail 130. The second rail 130 is moved onto the conically tapered face 108 opposite the conically tapered face 107 such that the protruding part of the plate 120 of the first end face 105 enters the slit like hole 138 of the second rail 135 and the protruding part of the plate 120 of the second end face 106 enters the slit like hole 139 of the second rail 135.

Second, the shielding box 100 together with the first rail 130 and the second rail 135 are moved towards the bushing 140 and the first rail 130 and the second rail 135 are fixed at the bushing 140 using screws 156.

Third, the first end 131 of the first rail 130 and the first end 136 of the second rail 135 are fixed to the first reinforcement structure 151 and the second reinforcement structure 152.

After that the second casing member may be fixed to the casing member 150 and the bushing 140. Before that step, tubes running from the bushing 140 towards the casing member 150 may also be connected.

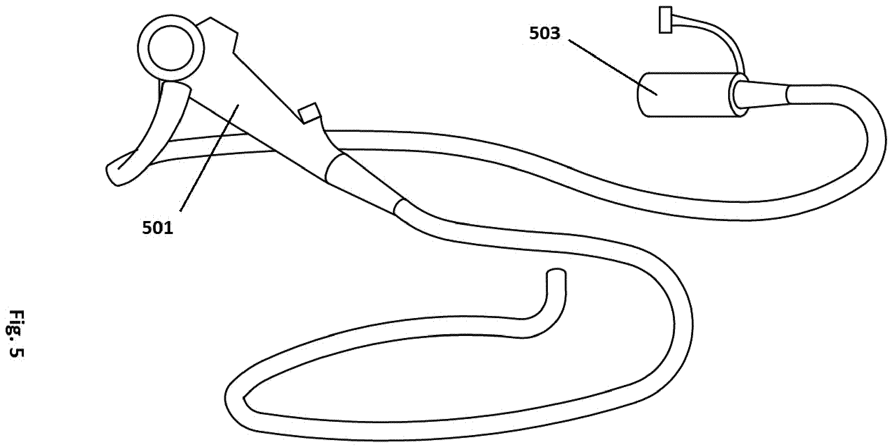

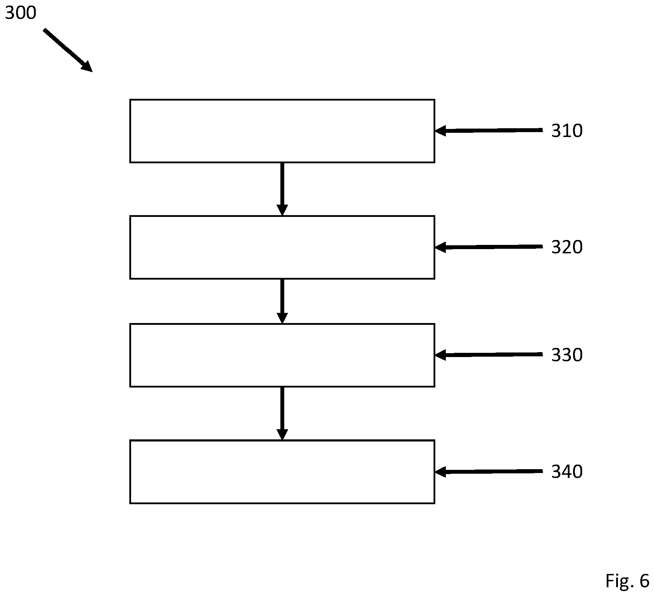

Figure 6 shows a flow chart for a method 300 for fixing the RF shielding box 100 inside the connector 200 for the endoscope 212 shown in figure 5. The shielding box 100 is the above mentioned shielding box 100.

According to a first step 310 of the method 300 a second end 132 of a first rail 130 and a second end 137 of a second rail 135 are fixed to opposing ends 141, 142 on an outside of an end side fixation device 140 of a cable 220 which is connectable to the connector 200.

According to a second step 320 of the method 300 the shielding box 100 is guided such that plates 120 of a second end face 106 run inside of elongated holes 145 of both the first rail 130 and the second rail 135 until the plates 120 contact an end of the elongated holes 145 near a second end 132, 137 of the first rail 130 or second rail 135.

According to a third step 330 a casing element 150 of the connector 200 is provided such that opposing ends 151, 152 of an outer rim 154 of an opening 153 of the casing element 150 are adjacent to a first end 131 of the first rail 130 and a first end 136 of the second rail 135.

According to a fourth step 340 the first end 131 of the first rail 130 and the first end 136 of the second rail 135 are fixed to the opposing ends 151, 152 on the outer rim 154 of the opening 153 of the casing element 150 of the connector 200.

## Claims

1. A radio-frequency , RF, shielding box (100) for use inside a connector (200) for a medical instrument (210), in particular an endoscope (212), wherein the shielding box (100) has a base area (102) which is conically tapered from a first end (103) of the shielding box (100) to a second end (104) of the shielding box (100); wherein both a first end face (105) and a second end face (106) of the shielding box (100) are rectangular shaped; wherein two rectangular shaped plates (120) protruding from the area of the first end face (105) and the second end face (106) are arranged each on the first end face (105) or on the second end face (106) of the shielding box (100), respectively, where the plates (120) are parallel to the first end face (105) or the second end face (106), respectively; and wherein the plates (120) of the first end face (105) and the second end face (106) of the shielding box (100) are parallel to each other, respectively; wherein the protruding parts of the plates (120) are adapted and installed such that the shielding box (100) can be mounted inside the connector (200) wherein at least two of the protruding parts of the plates (120) are adapted and installed to be arranged inside at least two holes (133, 134, 138, 139), wherein the holes (133, 134, 138, 139) are arranged inside a first and a second rail (130, 135) of the connector (200) which, when the shielding box (100) is mounted inside the connector (200), are parallel to conically tapered faces (107, 108) of the shielding box (100), and the connector (200) further comprising: the first rail (130) and the second rail (135); and wherein the first rail (130) and the second rail (135) each comprise the holes (133, 134, 138, 139) on a first end (131, 136) and a second end (132, 137) of the first rail (130) or the second rail (135); wherein the holes (133, 138) on the first end (131, 136) of the first rail (130) and the second rail (135) are adjacent to the first end face (105) of the shielding box (100) when the shielding box (100) is mounted inside the connector (200); and/or the holes (134, 139) on the second end (132, 137) of the first rail (130) and the second rail (135) are adjacent to the second end face (106) of the shielding box (100) when the shielding box (100) is mounted inside the connector (200).

2. Shielding box (100) according to claim 1, wherein longitudinal axes of the plates (120) each are parallel to the base area (102).

3. Shielding box (100) according to claim 1 or 2, wherein the base area (102) is an isosceles trapezoid.

4. Shielding box (100) according to any of the preceding claims, wherein the base area (102) and the two rectangular shaped plates (120) are part of a single workpiece.

5. Shielding box (100) according to any of the preceding claims, wherein the two protruding parts of the plate (120) of the first end face (105) are arranged inside the holes (133, 138) on the first end (131, 136) of the first rail (130) and the second rail (135) and/or the two protruding parts of the plate (120) of the second end face (106) are arranged inside the holes (134, 139) on the second end (132, 137) of the first rail (130) and the second rail (135).

6. Shielding box (100) according to any of the preceding claims, wherein the holes (133, 134, 138, 139) are slits.

7. Shielding box (100) according to any of the preceding claims, wherein the shielding box (100) is adapted and installed to be press fitted between the first and the second rail of the connector (200)

8. Shielding box (100) according to any of the preceding claims, wherein the first end (131) of the first rail (130) and the first end (136) of the second rail (135) are adapted to be fixed to opposing ends (151, 152) on an inside of a casing element (150) of the connector (200); and / or the second end (132) of the first rail (130) and the second end (137) of the second rail (135) are adapted to be fixed to opposing ends (141, 142) on the outside of an end side fixation device (140) of a cable (220) which is connectable to the connector (200).

9. Shielding box (100) according to claim 7, wherein the press fit is achieved by bending the first end (131) of the first rail (130) towards the second rail (135) and then fixing the first end (131) of the first rail (130) to an inside of a casing element (150); and/or by bending the first end (136) of the second rail (135) towards the first rail (130) and then fixing the first end (136) of the second rail (135) to an inside of the casing element (150).

10. Medical instrument (210), in particular an endoscope (212), comprising a shielding box (100) according to any of the preceding claims.

11. Method (300) for fixing a RF shielding box (100) inside a connector (200) for a medical instrument (210), in particular an endoscope (212), the shielding box (100) being a shielding box (100) according to any of claims 1 to 9, comprising: fixing (310) a second end (132) of a first rail (130) and a second end (137) of a second rail (135) to opposing ends (141, 142) on an outside of an end side fixation device (140) of a cable (220) which is connectable to the connector (200); guiding (320) the shielding box (100) such that the protruding parts of the plates (120) of a second end face (106) run inside of elongated holes (145) of both the first rail (130) and the second rail (135) until the plates (120) contact an end of the elongated holes (145) near a second end (132, 137) of the first rail (130) or second rail (135); providing (330) a casing element (150) of the connector (200) such that opposing ends (151, 152) of an outer rim (154) of an opening (153) of the casing element (150) are adjacent to a first end (131) of the first rail (130) and a first end (136) of the second rail (135); and fixing (340) the first end (131) of the first rail (130) and the first end (136) of the second rail (135) to the opposing ends (151, 152) on the outer rim (154) of the opening (153) of the casing element (150) of the connector (200).

## Patentansprüche

1. HF-Abschirmbox (100) zur Verwendung innerhalb eines Steckers (200) für ein medizinisches Instrument (210), insbesondere ein Endoskop (212), wobei die Abschirmbox (100) zylinderförmig ist, bei der es sich um eine dreidimensionale Form handelt, die durch eine zylindrische Oberfläche und zwei parallele Ebenen begrenzt ist, und eine Grundfläche (102) aufweist, die sich von einem ersten Ende (103) der Abschirmbox (100) zu einem zweiten Ende (104) der Abschirmbox (100) konisch verjüngt; wobei sowohl eine erste Stirnfläche (105) als auch eine zweite Stirnfläche (106) der Abschirmbox (100) rechteckig geformt sind; wobei zwei rechteckig geformte Platten (120), die aus dem Bereich der ersten Stirnfläche (105) und der zweiten Stirnfläche (106) hervorstehen, auf der ersten Stirnfläche (105) bzw. auf der zweiten Stirnfläche (106) der Abschirmbox (100) angeordnet sind, wobei die Platten (120) parallel zu der ersten Stirnfläche (105) bzw. zu der zweiten Stirnfläche (106) sind; und wobei die Platten (120) der ersten Stirnfläche (105) bzw. der zweiten Stirnfläche (106) der Abschirmbox (100) jeweils parallel zueinander sind; wobei die hervorstehenden Teile der Platten (120) so ausgelegt und installiert sind, dass die Abschirmbox (100) im Inneren des Verbinders (200) montiert werden kann, wobei mindestens zwei der hervorstehenden Teile der Platten (120) ausgelegt und installiert sind, um innerhalb von mindestens zwei Löchern (133, 134, 138, 139) angeordnet zu werden, wobei die Löcher (133, 134, 138, 139) innerhalb einer ersten und einer zweiten Schiene (131, 132) angeordnet sind, die bei bestimmungsgemäßer Verwendung jeweils parallel zu den sich konisch verjüngenden Flächen (107, 108) der Abschirmbox (100) sind, und wobei die Abschirmbox (100) ferner umfasst: eine erste Schiene (130) und eine zweite Schiene (135), die jeweils parallel zu sich konisch verjüngenden Flächen (107, 108) der Abschirmbox (100) sind; und wobei die erste Schiene (130) und die zweite Schiene (135) jeweils ein Loch (133, 134, 138, 139) an einem ersten Ende (131, 136) und an einem zweiten Ende (132, 137) der ersten Schiene (130) oder der zweiten Schiene (135) umfassen; wobei die Löcher (133, 138) an dem ersten Ende (131, 136) der ersten Schiene (130) und der zweiten Schiene (135) an die erste Stirnfläche (105) der Abschirmbox (100) angrenzen; und/oder die Löcher (134, 139) an dem zweiten Ende (132, 137) der ersten Schiene (130) und der zweiten Schiene (135) an die zweite Stirnfläche (106) der Abschirmbox (100) angrenzen.

2. Abschirmbox (100) nach Anspruch 1, wobei Längsachsen der Platten (120) jeweils parallel zu der Grundfläche (102) eines Zylinders (101) sind.

3. Abschirmbox (100) nach Anspruch 1 oder 2, wobei die Grundfläche (102) ein gleichschenkliges Trapez ist.

4. Abschirmbox (100) nach einem der vorhergehenden Ansprüche, wobei die Grundfläche (102) und die beiden rechteckig geformten Platten (120) Teil eines einzigen Werkstücks sind, das durch Biegen einer Schneidplatte an mindestens einer Kante hergestellt ist.

5. Abschirmbox (100) nach einem der vorhergehenden Ansprüche, wobei die beiden hervorstehenden Teile der ersten Stirnfläche (105) innerhalb der Löcher (133, 134, 138, 139) an dem ersten Ende (131, 136) der ersten Schiene (130) und der zweiten Schiene (135) angeordnet sind und/oder die beiden hervorstehenden Teile der zweiten Stirnfläche (106) innerhalb der Löcher (134, 139) an dem zweiten Ende (132, 137) der ersten Schiene (130) und der zweiten Schiene (135) angeordnet sind.

6. Abschirmbox (100) nach einem der vorhergehenden Ansprüche, wobei die Löcher (133, 134, 138, 139) Schlitze sind.

7. Abschirmbox (100) nach einem der vorhergehenden Ansprüche, wobei die Abschirmbox (100) ausgelegt und installiert ist, um zwischen der ersten und der zweiten Schiene eingepresst zu werden.

8. Abschirmbox (100) nach einem der vorhergehenden Ansprüche, wobei das erste Ende (131) der ersten Schiene (130) und das erste Ende (136) der zweiten Schiene (135) ausgelegt sind, um an gegenüberliegenden Enden (151, 152) an einer Innenseite eines Gehäuseelements (150) des Verbinders (200) befestigt zu werden; und/oder das zweite Ende (132) der ersten Schiene (130) und das zweite Ende (137) der zweiten Schiene (135) ausgelegt sind, um an gegenüberliegenden Enden (141, 142) an der Außenseite einer endseitigen Befestigungsvorrichtung (140) eines Kabels (220) befestigt zu werden, das mit dem Verbinder (200) verbindbar ist.

9. Abschirmbox (100) nach Anspruch 8, wobei die Presspassung durch Biegen des ersten Endes (131) der ersten Schiene (130) in Richtung der zweiten Schiene (135) und anschließendes Befestigen des ersten Endes (131) der ersten Schiene (130) an einer Innenseite eines Gehäuseelements (150); und/oder durch Biegen des ersten Endes (136) der zweiten Schiene (135) in Richtung der ersten Schiene (130) und anschließendes Befestigen des ersten Endes (136) der zweiten Schiene (135) an einer Innenseite des Gehäuseelements (150) erreicht wird.

10. Medizinisches Instrument (210), insbesondere Endoskop (212), das eine Abschirmbox (100) nach einem der vorhergehenden Ansprüche umfasst.

11. Verfahren (300) zum Befestigen einer HF-Abschirmbox (100) im Inneren eines Verbinders (200) für ein medizinisches Instrument (210), insbesondere ein Endoskop (212), wobei die Abschirmbox (100) vorzugsweise eine Abschirmbox (100) nach einem der Ansprüche 1 bis 9 ist, wobei das Verfahren umfasst: Befestigen (310) eines zweiten Endes (132) einer ersten Schiene (130) und eines zweiten Endes (137) einer zweiten Schiene (135) an gegenüberliegenden Enden (141, 142) an einer Außenseite einer endseitigen Befestigungsvorrichtung (140) eines Kabels (220), das mit dem Verbinder (200) verbindbar ist; Führen (320) der Abschirmbox (100), sodass Platten (120) einer zweiten Stirnfläche (106) innerhalb von Langlöchern (145) sowohl der ersten Schiene (130) als auch der zweiten Schiene (135) laufen, bis die Platten (120) ein Ende der Langlöcher (145) nahe einem zweiten Ende (132, 137) der ersten Schiene (130) oder der zweiten Schiene (135) berühren; Bereitstellen (330) eines Gehäuseelements (150) des Verbinders (200), sodass gegenüberliegende Enden (151, 152) eines äußeren Randes (154) einer Öffnung (153) des Gehäuseelements (150) einem ersten Ende (131) der ersten Schiene (130) und einem ersten Ende (136) der zweiten Schiene (135) benachbart sind; und Befestigen (340) des ersten Endes (131) der ersten Schiene (130) und des ersten Endes (136) der zweiten Schiene (135) an den gegenüberliegenden Enden (151, 152) an dem äußeren Rand (154) der Öffnung (153) des Gehäuseelements (150) des Verbinders (200).

## Revendications

1. Boîtier de blindage RF (100) destiné à être utilisé à l'intérieur d'un organe de liaison (200) pour un instrument médical (210), notamment un endoscope (212), dans lequel le boîtier de blindage (100) est de forme cylindrique, étant d'une forme tridimensionnelle délimitée par une surface cylindrique et deux plans parallèles, ayant une zone de base (102) qui est coniquement effilée depuis une première extrémité (103) du boîtier de blindage (100) jusqu'à une seconde extrémité (104) du boîtier de blindage (100) ; dans lequel à la fois une première face d'extrémité (105) et une seconde face d'extrémité (106) du boîtier de blindage (100) sont de forme rectangulaire ; dans lequel deux plaques de forme rectangulaire (120) faisant saillie de la zone de la première face d'extrémité (105) et de la seconde face d'extrémité (106) sont agencées sur la première face d'extrémité (105) ou sur la seconde face d'extrémité (106) du boîtier de blindage (100), respectivement, où les plaques (120) sont parallèles à la première face d'extrémité (105) ou à la seconde face d'extrémité (106), respectivement ; et dans lequel les plaques (120) de la première face d'extrémité (105) et de la seconde face d'extrémité (106) du boîtier de blindage (100) sont parallèles entre elles, respectivement ; dans lequel les parties saillantes des plaques (120) sont adaptées et installées de telle sorte que le boîtier de blindage (100) peut être monté à l'intérieur de l'organe de liaison (200), dans lequel au moins deux des parties saillantes des plaques (120) sont adaptées et installées pour être agencées à l'intérieur d'au moins deux trous (133, 134, 138, 139), dans lequel les trous (133, 134, 138, 139) sont agencés à l'intérieur d'un premier et d'un second rail (131, 132) qui, lorsqu'ils sont utilisés comme prévu, sont chacun parallèles aux faces coniquement effilées (107, 108) du boîtier de blindage (100), et le boîtier de blindage (100) comprenant en outre : un premier rail (130) et un second rail (135), qui sont chacun parallèles aux faces coniquement effilées (107, 108) du boîtier de blindage (100) ; et dans lequel le premier rail (130) et le second rail (135) comprennent chacun un trou (133, 134, 138, 139) sur une première extrémité (131, 136) et une seconde extrémité (132, 137) du premier rail (130) ou du second rail (135) ; dans lequel les trous (133, 138) sur la première extrémité (131, 136) du premier rail (130) et du second rail (135) sont adjacents à la première face d'extrémité (105) du boîtier de blindage (100) ; et/ou les trous (134, 139) sur la seconde extrémité (132, 137) du premier rail (130) et du second rail (135) sont adjacents à la seconde face d'extrémité (106) du boîtier de blindage (100).

2. Boîtier de blindage (100) selon la revendication 1, dans lequel les axes longitudinaux des plaques (120) sont chacun parallèles à la zone de base (102) d'un cylindre (101).

3. Boîtier de blindage (100) selon la revendication 1 ou 2, dans lequel la zone de base (102) est un trapèze isocèle.

4. Boîtier de blindage (100) selon l'une quelconque des revendications précédentes, dans lequel la zone de base (102) et les deux plaques de forme rectangulaire (120) font partie d'une seule pièce à usiner qui est fabriquée en pliant une plaque de coupe au niveau d'au moins un bord.

5. Boîtier de blindage (100) selon l'une quelconque des revendications précédentes, dans lequel les deux parties saillantes de la première face d'extrémité (105) sont agencées à l'intérieur des trous (133, 134, 138, 139) sur la première extrémité (131, 136) du premier rail (130) et du second rail (135) et/ou les deux parties saillantes de la seconde face d'extrémité (106) sont agencées à l'intérieur des trous (134, 139) sur la seconde extrémité (132, 137) du premier rail (130) et du second rail (135).

6. Boîtier de blindage (100) selon l'une quelconque des revendications précédentes, dans lequel les trous (133, 134, 138, 139) sont des fentes.

7. Boîtier de blindage (100) selon l'une quelconque des revendications précédentes, dans lequel le boîtier de blindage (100) est adapté et installé pour être ajusté par pression entre le premier et le second rail.

8. Boîtier de blindage (100) selon l'une quelconque des revendications précédentes, dans lequel la première extrémité (131) du premier rail (130) et la première extrémité (136) du second rail (135) sont adaptées pour être fixées à des extrémités opposées (151, 152) à l'intérieur d'un élément de coque (150) de l'organe de liaison (200) ; et/ou la seconde extrémité (132) du premier rail (130) et la seconde extrémité (137) du second rail (135) sont adaptées pour être fixées à des extrémités opposées (141, 142) à l'extérieur d'un dispositif de fixation côté extrémité (140) d'un câble (220) qui peut être relié à l'organe de liaison (200).

9. Boîtier de blindage (100) selon la revendication 8, dans lequel l'ajustement par pression est obtenu en pliant la première extrémité (131) du premier rail (130) vers le second rail (135) puis en fixant la première extrémité (131) du premier rail (130) à l'intérieur d'un élément de coque (150) ; et/ou en pliant la première extrémité (136) du second rail (135) vers le premier rail (130) puis en fixant la première extrémité (136) du second rail (135) à l'intérieur de l'élément de coque (150).

10. Instrument médical (210), notamment un endoscope (212), comprenant un boîtier de blindage (100) selon l'une quelconque des revendications précédentes.

11. Procédé (300) de fixation d'un boîtier de blindage RF (100) à l'intérieur d'un organe de liaison (200) pour un instrument médical (210), notamment un endoscope (212), le boîtier de blindage (100) étant de préférence un boîtier de blindage (100) selon l'une quelconque des revendications 1 à 9, comprenant : la fixation (310) d'une seconde extrémité (132) d'un premier rail (130) et d'une seconde extrémité (137) d'un second rail (135) à des extrémités opposées (141, 142) sur l'extérieur d'un dispositif de fixation côté extrémité (140) d'un câble (220) qui peut être relié à l'organe de liaison (200) ; le guidage (320) du boîtier de blindage (100) de telle sorte que des plaques (120) d'une seconde face d'extrémité (106) passent à l'intérieur de trous allongés (145) à la fois du premier rail (130) et du second rail (135) jusqu'à ce que les plaques (120) entrent en contact avec une extrémité des trous allongés (145) à proximité d'une seconde extrémité (132, 137) du premier rail (130) ou du second rail (135) ; la fourniture (330) d'un élément de coque (150) de l'organe de liaison (200) de telle sorte que des extrémités opposées (151, 152) d'un bord externe (154) d'une ouverture (153) de l'élément de coque (150) sont adjacentes à une première extrémité (131) du premier rail (130) et à une première extrémité (136) du second rail (135) ; et la fixation (340) de la première extrémité (131) du premier rail (130) et de la première extrémité (136) du second rail (135) aux extrémités opposées (151, 152) sur le bord externe (154) de l'ouverture (153) de l'élément de coque (150) de l'organe de liaison (200).
